# EUROPEAN PATENT SPECIFICATION

(11) **EP 0 983 075 B1**
(45) Date of publication and mention of the grant of the patent: **21.08.2002**
(21) Application number: 98924669.9
(22) Date of filing: 25.05.1998
(51) Int. Cl.: A61K 35/407, C12N 5/06, C12N 5/08, C12Q 1/00

(54) **A METHOD TO INCREASE THE SURVIVAL OF TRANSPLANTED CELLS**
METHODE ZUR STEIGERUNG DER ÜBERLEBUNG TRANSPLANTIERTER ZELLEN
PROCEDE SERVANT A AUGMENTER LA SURVIE DE CELLULES TRANSPLANTEES

(30) Priority: 21.05.1997 EP 97201514
(43) Date of publication of application: 08.03.2000
(73) Proprietor: Leadd B.V., 2333 AL Leiden (NL)
(72) Inventor: BRUIN, Jan, Anthonie, NL-2243 EE Wassenaar (NL); TERPSTRA, Onno, NL-2649 SC Pijnacker (NL)
(74) Representative: Ottevangers, Sietse Ulbe
(86) International application number: NL9800292
(87) International publication number: WO98052584

(56) References cited:
- WO-A-95/27042
- D. FAUSTMAN ET AL.: "PREVENTION OF XENOGRAFT REJECTION BY MASKING DONOR HLA CLASS I ANTIGENS." SCIENCE, vol. 252, 21 June 1991, pages 1700-1702, XP002011037 LANCASTER, PA US
- S.C. STAMATOGLOU ET AL.: "AFFINITY OF INTEGRIN ALPHA1 BETA2 FROM LIVER SINUSOIDAL MEMBRANES FOR TYPE IV COLLAGEN." FEBS LETTERS, vol. 288, no. 1,2, August 1991, pages 241-243, XP002044821 AMSTERDAM NL
- J.M. KOCKEN ET AL.: "CORRECTION OF AN INBORN ERROR OF METABOLISM BY INTRAPORTAL HEPATOCYTE TRANSPLANTATION IN A DOG MODEL." TRANSPLANTATION, vol. 62, no. 3, 15 August 1996, pages 358-364, XP002044822 BALTIMORE, MD, US
- J.M. KOCKEN ET AL.: "BLOCKING OF ALPHA1 BETA2 INTEGRIN STRONGLY IMPROVES SURVIVAL OF HEPATOCYTES IN ALLOGENEIC TRANSPLANTATION." LABORATORY INVESTIGATION, vol. 77, no. 1, July 1997, pages 19-28, XP002077427 BALTIMORE, MD, US

## Description

The invention relates to the field of transplantation, specifically to the field of transplantation of isolated cells.

Total organ transplantation suffers from significant operative risks, complications, and insufficient donor supply. It would therefore be beneficial to be able to replace (parts of) the defunct organ by isolated cells, which can be administered easily, and in essence can provide similar relief to a patient as a fully transplanted organ could do. Where possible, total organ transplantation would therefore be favorably replaced by isolated cell transplantation. One approach is to transplant normal allogeneic cells from healthy individuals into the spleen or the portal vein of immunosuppressed recipients, without needing genetic modification of the cells. Another approach is to transplant genetically modified (via gene transfer techniques or the like) cells, being syngeneic or allogeneic, thereby replacing or supplementing cells with a genetic disorder by properly functioning cells.

For example, a pancreas lacking in pancreatic islets cells could be made functioning again by placing pancreatic islet cells within the recipient tissue of the defunct pancreas, or within other suitable recipient tissue. Also, for example in patients with inborn errors of liver-based metabolism, transplantation of isolated hepatocytes may be an attractive alternative to total liver replacement.

Total liver replacement as treatment for patients with hereditary enzyme deficiencies suffers from significant operative risks, complications, and insufficient donor supply. Interest in improving the transplantation of isolated hepatocytes has therefore increased in recent years. One approach is to transplant normal allogeneic hepatocytes from healthy individuals into the spleen or the portal vein of immunosuppressed recipients, without needing genetic modification of the cells. However, especially in the case of hereditary disorders, transplantation with genetically modified cells would be also very beneficial. Advantages of this technique are its simplicity and low cost. However, a major drawback of allogeneic hepatocyte transplantation (HTX) is the lack of sustained survival of the transplanted cells in the liver parenchyma of the recipient, and the need for immunosuppression. Previous studies in our laboratory demonstrated that strong immunosuppression improves cell survival two days after allogeneic HTX in rat models, but still most of the transplanted hepatocytes had disappeared ten days after HTX. Furthermore, large intravascular aggregates of transplanted hepatocytes were demonstrated in the liver in this study. Previous HTX studies in dogs also demonstrated intravascular hepatocyte aggregation in the recipient livers after allogeneic intraportal HIX. Further studies showed, that the intravascular hepatocyte aggregates contain both fibrin and thrombocytes, suggesting involvement of the coagulation system. However, heparinisation of recipient rats did not prevent the intravascular aggregation of transplanted hepatocytes. Experimental work in this field has involved both syngeneic and allogeneic transplantation. Previous studies on syngeneic hepatocyte transplantation (HTX) demonstrated intraparenchymal survival of 14% of transplanted hepatocytes upon intraportal administration, while no intravascular aggregation was observed. In allogeneic HTX one of the major problems encountered is the formation of intravascular hepatocyte aggregates soon after intraportal HTX. Clearly, intravascular aggregation upon injection hampers the entrance of the cells into the liver parenchyma and may explain the lower intraparenchymal cell survival observed after allogeneic HTX, when compared to that after syngeneic HTX. Within the cell aggregates fibrin and thrombocytes are present, indicating activation of the coagulation cascade. However, heparin treatment does not prevent the aggregation, suggesting the involvement of adhesion mechanisms other than, or in addition to coagulation.

The invention now provides a method to prevent or diminish intra-vascular aggregation of transplanted cells by pre-treating the cells with substances that functionally (transiently) block cell adhesion molecules or integrins. The cells to be transplanted can be isolated cells obtained from organ tissue, but can also comprise (small) structural units of cells such as pancreatic islets. In general, two types of cell adhesion exist: cell-extracellular matrix adhesion and cell-cell adhesion. Cells adhere to other cells or to the extracellular matrix via specific cell surface receptors, most of which belong to a family of structurally related glycoproteins called integrins. The composition of integrins expressed by different cell types varies greatly. Previous studies in rats have demonstrated that integrin molecules play a role in cell-cell and cell-matrix interactions in the liver. It is now found that integrin mediated interactions are responsible for the observed intravascular aggegration of transplanted cells, and prevent their migration into the recipient tissue. In addition to the method provided by the invention which diminishes cell-adhesion and thereby decreases intravascular aggregation the invention provides a method to increase homing of cells in the matrix of the recipient tissue and provides a method to increase the survival of transplanted cells. The invention also provides a method wherein the cells are first genetically modified, hereby enabling the transplantation of cells specifically equipped to help cure or prevent a genetic disorder. A preferred embodiment of the invention entails the use of allogeneic cells. Another possible embodiment of the invention entails the use of xenogeneic cells, for example pancreatic islets, isolated from for example a pig, for transplantation to diabetes patients. An example of a method provided by the invention is given in the experimental part, wherein the cells are liver cells or hepatocytes. The experimental part illustrates a method wherein the recipient tissue is the liver. Furthermore, a preferred embodiment of the invention is a method wherein the wherein the integrin is α1β1 integrin. The invention provides a method wherein the blocking substance can be any substance (transiently) binding to integrin. This can for example be a carbohydrate or a (poly)peptide. A preferred embodiment of a method provided by the invention is a method where the blocking substance is an antibody or fragment thereof. Especially binding peptides, which can for example be obtained with classical PEPSCAN methods using integrin as test substrate, are suitable for use in a method provided by the invention. The invention provides a distinct advantage for use in cell transplantation, preferably in allogeneic cell transplantation.

Also, the invention provides an *in vitro* method to select blocking substances or (poly)peptides for their capacity to diminish cell-cell adhesion or cell-extracellular matrix adhesion. As an example the invention provides a method by which hepatocyte attachment can be studied in an *in vitro* binding assay on cryostat sections of liver tissue using a panel of tentative blocking substances or (poly)peptides. The invention thus provides a method to select a relevant blocking substance, and thereby provides a relevant blocking substance. With relevant blocking substances, as identified above or obtained by a method above, a pharmaceutical composition can be prepared for the pre-treatment of cells. This composition contains a relevant blocking substance which diminishes cell adhesion and a suitable diluent, which can be selected from the many diluents fit for transplantation purposes known in the art.

### Experimental part

The experimental part describes a method according to the invention which is related to liver cell transplantation, however, the experimental part can in no way be seen as limiting the invention.

### Materials and methods

### In vitro experiments

### Isolation of hepatocytes

Brown Norway (BN) hepatocytes were isolated using a modification of the Seglen collagenase digestion technique. Briefly, after cannulation of the portal vein, the liver was perfused with Hepes-based buffer (Boehringer Mannheim, The Netherlands) adjusted to pH 7.4 (10 mM hepes; 142 mM NaCl; 6.7 mM KCI) at a flow rate of 30 ml/min. until the perfusate was free of erythrocytes. Then recirculation was started for 10-15 min. with Hepes buffered at pH 7.6 (100 mM Hepes; 56.7 mM NaCl; 5mM CaCI2.2H20) containing 0.5 mg/ml collagenase I (Sigma, Chemicals Co., St. Louis,M0,USA). The hepatocyte suspension was filtered (40 µm sieve) and centrifuged at 50xg for 6 min. After gentle resuspension with Hanks balanced salt solution (HBSS) the hepatocytes were counted and viability was determined by Trypan blue exclusion (0.02%). Finally, the freshly isolated hepatocytes were resuspended at a concentration of 10⁶ viable cells/ml in HBSS, and kept on ice until used.

### Antibodies

Function blocking monoclonal antibody against CD29, the common β1 integrin subunit of the VLA sub-family, was purchased from the Central Laboratory of Blood transfusion (Amsterdam, The Netherlands); mabs against α1 integrin (CD49a), and against the integrin α2 chain (CD49b) were purchased from Pharmingen (Palo Alto, CA); mabs against α4 and α6 integrins were generous gifts from Dr. E. Ruoslahti, La Jolla, California; Hybridomas against rat β2 (CD18, clone WT-3), rat LFA-Iα (CD11a, clone WT-1), and rat ICAM-I (CD54, clone IA29) were all generous gifts from Dr. M. Miysaka, Tokyo Metropolitan Institute of Medical Science, Tokyo, Japan; For isotypematched irrelevant mabs we used hamster mAb 4C7 against γδ-TCR (Pharmingen, Palo Alto, CA) and mouse IgG I mAb G250 against human renal cell carcinoma. Culture supernatant of hybridoma OX27, recognizing RT-IAⁿ (obtained from ECACC, Porton Down, Salisbury, UK), was used as an isotype-specific control antibody.

### Integrin expression

Expression of the various integrins was determined using an immunoperoxidase staining technique on BN hepatocyte cytospins, normal Lewis liver sections, and Lewis liver sections of recipient rats at day 2 after HTX of BN hepatocytes, containing hepatocyte aggregates. Secondary mabs alone or PBS as a first step were used as negative controls and culture supernatant of hybridoma OX27 (against MHC class I), as a positive control. N=3 in each group.

### IN VITRO detection of suitable binding or blocking (poly)peptides

### BN hepatocyte attachment to Lewis liver sections in vitro

The *in vitro* binding was evaluated of a freshly isolated, single cell BN hepatocyte suspension to fresh, unfixed liver tissue sections, obtained from untreated male Lewis rats. Three biopsies, one each from three different liver lobes of a Lewis rat, were snap-frozen in isopentane (2-methylbutane, Merck, Damstadt, Germany) and stored at -70 OC. Cryostat sections, 5 µm thick, were air dried for 1-2 hours. BN hepatocytes were isolated as outlined above. From a hepatocyte suspension of 1 x 10⁶ viable cells/ml 80µl was pipetted on liver cryostat sections. The glass slides were incubated at 37 °C, 5 % C02, 95% air for 30, 60, or 90 min. respectively. The slides were washed three times with PBS to remove unattached cells and then fixed in 4% paraformaldehyde in PBS for 30 min. The attached BN hepatocytes were visualized by a three step immunoperoxidase technique using anti-MHC-1 mouse monoclonal OX27 as the first step. A peroxidase-labeled rabbit anti-mouse antibody was used as second step antibody (Dako, Glostrup, Denmark), and a peroxidase-labeled swine anti-rabbit antibody as a third-step (Dako, Glostrup, Derunark).

For every separate anti-integrin mAb the number of attached hepatocytes to vessel walls in the cryostat sections was determined in 15 randomly chosen fields of each 1 cm². The number of hepatocytes attached to the liver parenchyma was counted in 15 other randomly chosen fields of 1 square cm, in which more than 90% of the grid was liver parenchyma. In a control group the numbers of cells attached to vessel walls and to liver parenchyma without pre-incubation with anti-integrin mAb were determined. The mean numbers of attached cells for separate anti-integrin mAbs are presented as percentages of the numbers in the control groups at the same time points (Table 1).

### Inhibition or blocking of hepatocyte attachment to liver sections in vitro by function-blocking mAbs.

A hepatocyte suspension of I x IO⁶ viable BN cells per ml was incubated for 10 min. at 37°C with function-blocking monoclonal antibodies (mAbs) in concentrations of 40µg/ml. The hepatocyte-mab suspensions were then plated on fresh Lewis cryostat liver sections as described above. Three sections, one each from three different liver lobes, were incubated for 30, 60, and 90 min. and then washed, fixed, stained, and counted as outlined above. Control sections were incubated with isotype-matched irrelevant mabs in the same concentration.

### IN VIVO experiments

### Animals

Adult male BN rats (RT-IAⁿ) and adult male Lewis rats (RT-IA¹) were purchased from Charles River (Broekman Institute, Someren, The Netherlands) and were between 150-200 g body weight when used. Rats were kept under standard laboratory conditions with 12/12-hr dark/light rhythm and standard pallet food. Surgical procedures were performed under ether anesthesia. AR experiments were carried out in compliance with the guidelines on animal well fare of the National Veterinary Inspection, The Netherlands.

### Immune suppression of the recipients

Recipient Lewis rats were treated with a combination of 5 Gy total body irradiation (TBI) on day -2 and Cyclosporine-A (CsA, Sandoz Pharmaceutical Corp., Basel, Switzerland) administration on days -2, -1, 0 -(25 mg/kg, i.m.).

### Hepatocyte transplantation

Transplantation was performed via midline laparotomy, followed by injection of a I ml single cell suspension containing 1 x 10⁷ viable hepatocytes into the portal vein of the recipient rats (0.33 mm x 13 mm needle). Leakage of cell suspension or blood was minimized by tamponade of the injection site with a cotton tip.

### Experimental design

Intraportal HTX of BN hepatocytes was performed using immunosuppressed Lewis rats as recipients. Donor hepatocytes were incubated with the following antibodies at a concentration of 200µg/ml. As in the *in vitro* experiments 1 x 10⁶ viable cells/nd were used, and in the *in vivo* experiments 1 x 10⁷ ( i.e. 10-fold more).

The experimental groups were subdivided as follows:
Group A: no pre-incubation with immunoglobulins or blocking polypeptides (n= 6).
Group B: pre-incubation with blocking anti-α1 integrin mAb (n=4).
Group C: pre-incubation with blocking anti-β1 integrin mAb (n=4).
Group D: pre-incubation with blocking anti-LFA-α1 integrin mAb (n = 4).
Group E: pre-incubation with blocking anti-β2 integrin mAb (n=4).
Group F: pre-incubation with normal mouse immunoglobulins (n=5).

Recipient rats were sacrificed at day 2 after HTX.

### Evaluation

For histological examination and immunohistochemistry, 6 liver biopsies, two each from 3 different liver lobes, were taken from each recipient rat. Previous studies in our laboratory have shown that hepatocytes are randomly distributed in the liver after HTX. For evaluation of the number of vessels containing hepatocyte aggregates, liver biopsies were fixed in 3.6% buffered formalin and embedded in paraffin. The number of hepatocyte aggregates was blindly scored in sections from 3 different slides from 3 different liver lobes in the recipient liver, after staining with hematoxylin and eosin and phosphotungstic acid haematoxilin (PTAH) procedure, and expressed per square mm. For quantitation of the percentage surviving transplanted hepatocytes found in the recipient livers, three biopsies were snap-frozen in isopentane. The percentage survival of the transplanted
hepatocytes was assessed at day 2 after HTX, in frozen sections using a three step immunoperoxidase technique as outlined above with mAb OX27. The number of donor hepatocytes located in the liver parenchyma was counted according to a previously described method.

### Statistical analysis

Statistical analysis was performed using the Krusskall-Wallis test, followed by the Mann-Whitney U test to evaluate the differences (considered significant when p<0.05) between groups; Analysis of Variance was applied using Scheffe's procedure and the LSD (least significant difference) test (considered significant when p<0.05). Values are expressed as mean SEM or as mean t SD.

### RESULTS

### IN VITRO experiments

### Differential expression of integrin on isolated hepatocytes, liver sections, and hepatocyte aggregates after HTX

The expression of MHC-1, β1, α1, α2, α4, α6, LFA-1α, β2, and ICAM-1 was analyzed by indirect immunoperoxidase staining and revealed that all tested integrins could be demonstrated on BN cytospins. The β1 integrin subunit and MHC-1 demonstrated the highest expression on isolated BN hepatocytes. However, on normal Lewis liver sections only LFA-1α and β2 could be demonstrated. On intravascular hepatocyte aggregates in livers of Lewis recipients at day 2 after HTX α4, ICAM-1, LFA-1α and β2 could be detected.

### BN hepatocyte attachment to Lewis liver sections in vitro

Hepatocytes exhibited equal attachment to cryostat sections of various liver lobes. Furthermore, attachment was time-dependent between 30 and 90 min., with maximal attachment obtained after 90 minutes incubation. Although the hepatocytes were applied to liver sections in at least 100-fold excess, more hepatocytes were found to be attached to vessel walls than to liver parenchyma. No hepatocytes were attached to the surrounding glass surface.

### Antibody-mediated inhibition of hepatocyte attachment to Lewis liver sections in vitro

In order to investigate the role of integrins and to detect blocking substances capable of blocking the attachment of hepatocytes to Lewis liver sections, we conducted experiments to inhibit the attachment using function-blocking anti-integrin mAbs. Hepatocyte binding to the cryostat sections was examined after 30, 60, and 90 min. incubation. Function blocking mAbs showed a very strong and significant (p<0.05) inhibition of attachment at t= 30, 60, and 90 min. to both vessel walls and parenchyma. Isotype specific control mabs did not influence binding of the hepatocytes to the liver sections tested. Similarly, no inhibition was obtained after incubation with the haplotype-specific antibody against Brown Norway MHC-I (OX27) when compared to attachment of hepatocytes without pre-incubation. Marked binding of hepatocytes to vessels and parenchyma was seen without mAb or with the unrelated mAb whereas binding was completely blocked after incubation with anti-β1 and with anti-α1 mabs.

### In vivo experiments

### Viability

During the hepatocyte infusion no major complications or mortality were encountered. The viability of the infused hepatocyte suspensions ranged from 85%- 98% in all groups.

### Hepatocyte survival

In group A (no incubation with anti-integrin blocking polypeptides) 2.39 +/- 0.27% of the transplanted hepatocytes was found in the liver parenchyma, which is not significantly different from the survival in group F (incubation with aspecific normal mouse immunoglobulins). In groups B, C and D resp. 44.77 +/- 8.49 %, 36.31 +/- 8.15% and 19.13 +/- 1.64% of the transplanted hepatocytes were found in the recipient liver at day 2 after HTX, which is significantly higher than the percentage in control groups A and F (p<0.001). Viability of these cells was determined by their morphology and membranous expression of MHC-I molecules.

In all groups, intravascular aggregates, consisting of transplanted hepatocytes and fibrin were found in portal veins and small venules in the recipient livers at day 2: In groups D and E the number of hepatocyte aggregates was significantly lower than in control group F (p<0.001).

### Morphology and histology

Microscopic examination of the livers in all groups showed intravascular aggregates consisting of plugs of transplanted BN hepatocytes surrounded by fibrin after PTAH staining. However, after incubation with blocking anti-integrin polypeptide, only a few aggregates were seen, and only in the center of the lumen of the vessels instead of in contact with the vessel wall, as was seen in groups A and F.

### DISCUSSION

In our *in vitro* study, attachment of virtually all hepatocytes to both vessel walls and parenchyma was blocked most efficiently and in a sustained manner after administration of function-blocking integrin mabs. Unrelevant mabs and even the haplotype-specific mAb OX27 showed no blocking of the attachment, confirming the specificity of the observed blocking by the anti-integrin mabs.

In a first experiment, the anti-integrin mAb, which appeared to induce the most significant and persistent binding depletion *in vitro,* was incubated with BN hepatocytes before HTX.

Upon subsequent injection of these hepatocytes
into two Lewis rats, virtually no intravascular aggregation occurred, while intraparenchymal hepatocyte survival at day 2 after HTX was significantly higher than that after injection of non-preincubated hepatocytes.

This demonstrates for the first time intraparenchymal survival of as much as 45% of transplanted hepatocytes at day 2 after HTX, due to an effect of integrin blocking.

Treatment with anti-integrin mAbs significantly decreased the number of hepatocyte aggregates, has a strong effect on hepatocyte survival in the liver parenchyma, and has a strong effect effect on intraparenchymal survival.

Also, the treatment alters the affinity of other adhesion molecules on the hepatocyte surface, which promote migration into the liver parenchyma.

Anti-integrin (poly)peptides significantly block attachment of allogeneic cells to both vessel walls and tissue in an *in vitro* binding assay. Blocking of integrins by incubation with blocking (poly)peptides significantly decreases the number of cellular aggregates after cell transplantation, and has a significant increasing effect on ccellular homing and survival in the recipient tissue. Blocking of integrin strongly improves transplanted cell survival in the recipient tissue after transplantation.

In short, survival of cells, such as hepatocytes, after transplantation is generally low, due to formation of intravascular cellular aggregates. As an example of the invention, we determined the role of integrins in intravascular aggregation and intraparenchymal survival of transplanted hepatocytes in a fully allogeneic rat model. First, the expression profile of various integrins was determined on both isolated hepatocytes *in vitro* and hepatocyte aggregates in recipient livers after intraportal transplantation of allogeneic hepatocytes. Next, the role of these integrins in hepatocyte aggregation was determined in an *in vitro* attachment assay on liver sections with function-blocking anti-integrin monoclonal antibodies (mAbs). The results showed that anti-α1β1 integrin mAbs significantly block hepatocyte attachment to vessel wals and liver parenchyma *in vitro.* Subsequently, the effects of pre-incubation of hepatocytes with anti-integrin mabs on their intravascular aggregation and on intraparenchymal survival was studied in an allogeneic HTX model. Pre-incubation with anti-LFA-1α or anti-β2 mAbs significantly inhibited intravascular hepatocyte aggregation, and anti-LFA-1α mAb enhanced intraparenchymal survival. Pre-incubation with anti-1α or anti-β2 mAbs did not inhibit aggregation but significantly improved survival from 2% to up to 45% at day 2 after transplantation (p<0.001). In conclusion, blocking of integrin significantly improves survival of allo-transplanted hepatocytes.

**Table 1.**

| Inhibition of hepatocyte attachment to cryostat section of Lewis liver lobes by mAbs against integrin chains | | | | | | |
|---|---|---|---|---|---|---|
| *attachement* | *vessel* | | | *parenchyma* | | |
| time | 30 min | 60 min | 90 min | 30 min | 60 min | 90 min |
| no blocking | 100 | 100 | 100 | 100 | 100 | 100 |
| 4C7 | 99.8 ± 6.3 | 111 ± 5 | 92 ± 6.5 | 123 ± 5.9 | 141.5 ± 8.5 | 96.75 ± 5.3 |
| G250 | 121 ± 0.1 | 119 ± 0.6 | 100 ± 0.9 | 129 ± 1 | 119 ± 0.9 | 94.5 ± 3.1 |
| β1 | 3.3 ± 1.1* | 7.6 ± 3.0* | 6.9 ± 2.0* | 2.18 ± 0.7* | 7.0 ± 3.7* | 5.8 ± 1.0* |
| α1 | 12.8 ± 5.3* | 12.3 ± 2.3* | 16.1 ± 2.7* | 3.55 ± 1.3* | 5.1 ± 1.8* | 3.8 ± 1.9* |
| α2 | 12.2 ± 2.9* | 36.6 ± 10.7* | 35.9 ± 11.0* | 7.1 ± 1.5* | 12.7 ± 6.6* | 16.9 ± 4,4* |
| α4 | 40.9 ± 12* | 66.3 ± 13.6 | 76.8 ± 6.4 | 75.2 ± 23.7 | 95.7 ± 23.9 | 98.6 ± 12.1 |
| α6 | 44.3 ± 9.8* | 60.6 ± 7.5 | 56.6 ± 8.0 | 43 ± 10.2* | 64.6 ± 14.1 | 53.7 ± 6.3 |
| β2 | 10.5 ± 1.5* | 95.4 ± 11.2 | 93.2 ± 11.1 | 5.4 ± 1.0* | 63.0 ± 4.3 | 89.6 ± 18.3 |
| LFA-1α | 30.2 ± 6.1* | 47.6 ± 12.5 | 41.7 ± 8.3 | 54.3 ± 21.6 | 63.1 ± 24.8 | 60.6 ± 11.9 |

| | | | | | | |
|---|---|---|---|---|---|---|
| N=3 for all groups. All values are given as a percentage of hepatocyte attachment without blocking at the same time point, in mean ± SEM. In all experiments an antibody concentration of 40 µg/ml was used. 4C7; control hamster mAb (anti-rat IgG) G250; control mouse IgG1mAb. * significantly different from no blocking (P<0.05), according to the Scheffe test. | | | | | | |

## Claims

1. A method comprising treating cells prior to transplantation with a substance that binds to integrin, resulting in to decreased intravascular aggregation and/or increased homing and/or increased survival of transplanted cells in recipient tissue.

2. A method according to claim 1 wherein the cells are genetically modified.

3. A method according to claims 1 or 2 wherein the cells are allogeneic or xenogeneic.

4. A method according to any of claims 1 to 3 wherein the cells are liver cells.

5. A method according to any of claims 1 to 4 wherein the recipient tissue is liver.

6. A method according to any of claims 1 to 5 wherein the integrin is α1β1 integrin.

7. A method according to any of claims 1 to 6 wherein the substance is a (poly)peptide.

8. A method according to claim 7 wherein the substance is an antibody or fragment thereof.

9. A method to select a suitable substance for use in a method according to any of claims 1 to 8 comprising testing a substance *in vitro* for its capacity to diminish cell-cell adhesion or cell-extracellular matrix adhesion.

10. A method according to claim 9 wherein said substance is tested using cryostat tissue sections.

11. Use of a substance selected by a method according to claim 9 or 10 in the treatment of cells to be transplanted in recipient tissue to decrease intravascular aggregation and/or increase homing and/or increase the survival of transplanted cells.

12. A composition for carrying out a method according to any of claims 1 to 11 comprising cells and a substance which diminishes cell adhesion, additionally comprising a suitable diluent.

13. Use of a substance which diminishes cell adhesion for the preparation of a pharmaceutical composition for pre-treatment of cells to be transplanted to decrease intravascular aggregation and/or increase the homing and/or increase survival of transplanted cells in recipient tissue.

14. Use of a substance which diminishes cell adhesion, selected by a method according to claim 9 or 10, for the preparation of a pharmaceutical composition for pre-treatment of cells to be transplanted to decrease intravascular aggregation and/or increase the homing and/or increase survival of transplanted cells in recipient tissue.

15. Use of a substance according to claim 13 or 14 wherein said cells to be transplanted are allogeneic or xenogeneic.

## Patentansprüche

1. Verfahren, bei dem Zellen vor einer Transplantation mit einer Substanz behandelt werden, die sich an Integrin bindet, zum Reduzieren intravaskulärer Aggregation und/oder zum Erhöhen des Homing-Verhaltens und/oder zum Erhöhen des Überlebens transplantierter Zellen im Empfängergewebe.

2. Verfahren nach Anspruch 1, bei dem die Zellen genetisch modifiziert sind.

3. Verfahren nach Anspruch 1, bei dem die Zellen allogen oder xenogen sind.

4. Verfahren nach einem der Ansprüche 1 bis 3, bei dem die Zellen Leberzellen sind.

5. Verfahren nach einem der Ansprüche 1 bis 4, bei dem das Empfängergewebe die Leber ist.

6. Verfahren nach einem der Ansprüche 1 bis 5, bei dem das Integrin α1β1-Integrin ist.

7. Verfahren nach einem der Ansprüche 1 bis 6, bei dem die Substanz ein Polypeptid ist.

8. Verfahren nach einem der Ansprüche 1 bis 7, bei dem die Substanz ein Antikörper oder ein Fragment desselben ist.

9. Verfahren zum Auswählen einer geeigneten Substanz zur Verwendung in einem Verfahren nach einem der Ansprüche 1 bis 8, bei dem eine Substanz *in vitro* auf ihre Kapazität zur Reduzierung der Adhäsion zwischen Zelle und Zelle oder der Adhäsion zwischen Zelle und Extrazellulär-Matrix getestet wird.

10. Verfahren nach Anspruch 9, bei dem die Substanz unter Verwendung von Cryostat-Gewebeabschnitten getestet wird.

11. Verwendung einer durch ein Verfahren nach Anspruch 9 oder 10 gewählten Substanz bei der Behandlung von in ein Empfängergewebe zu transplantierenden Zellen zum Reduzieren intravaskulärer Aggregation und/oder zum Erhöhen des Homing-Verhaltens und/oder zum Erhöhen des Überlebens transplantierter Zellen.

12. Zusammensetzung zum Ausführen eines Verfahrens nach einem der Ansprüche 1 bis 11, mit Zellen und einer Zell-Adhäsion verhindernden Substanz, zusätzlich mit einem geeigneten Verdünnungsmittel.

13. Verwendung einer die Zell-Adhäsion reduzierenden Substanz für die Zubereitung einer pharmazeutischen Zusammensetzung zur Vorbehandlung zu transplantierender Zellen zum Reduzieren intravaskulärer Aggregation und/oder zum Erhöhen des Homing-Verhaltens und/oder zum Erhöhen des Überlebens transplantierter Zellen im Empfängergewebe.

14. Verwendung einer gemäß einem Verfahren nach Anspruch 9 oder 10 gewählten, die Zell-Adhäsion reduzierenden Substanz für die Zubereitung einer pharmazeutischen Zusammensetzung zur Vorbehandlung zu transplantierender Zellen zum Reduzieren intravaskulärer Aggregation und/oder zum Erhöhen des Homing-Verhaltens und/oder zum Erhöhen des Überlebens transplantierter Zellen im Empfängergewebe.

15. Verwendung einer Substanz nach Anspruch 13 oder 14, bei dem die zu transplantierenden Zellen allogen oder xenogen sind.

## Revendications

1. Procédé comprenant l'étape consistant à traiter des cellules, avant transplantation, avec une substance qui se fixe à l'intégrine ayant pour résultat de diminuer l'agrégation intravasculaire et/ou d'augmenter le *homing* (migration vers le tissu receveur) et/ou d'augmenter la survie des cellules transplantées.

2. Procédé selon la revendication 1, dans lequel les cellules sont modifiées génétiquement.

3. Procédé selon la revendication 1 ou 2, dans lequel les cellules sont allogéniques ou xénogéniques.

4. Procédé selon l'une quelconque des revendications 1 à 3, dans lequel les cellules sont des cellules hépatiques.

5. Procédé selon l'une quelconque des revendications 1 à 4, dans lequel le tissu receveur est le foie.

6. Procédé selon l'une quelconque des revendications 1 à 5, dans lequel l'intégrine est l'α1β1-intégrine.

7. Procédé selon l'une quelconque des revendications 1 à 6, dans lequel la substance est un (poly)peptide.

8. Procédé selon la revendication 7, dans lequel la substance est un anticorps ou fragment de celui-ci.

9. Procédé de sélection d'une substance convenable destinée à être utilisée dans un procédé selon l'une quelconque des revendications 1 à 8 comprenant l'étape consistant à tester une substance *in vitro* pour évaluer sa capacité à diminuer l'adhérence cellule-cellule ou l'adhérence cellule-matrice extracellulaire.

10. Procédé selon la revendication 9, dans lequel ladite substance est testée en utilisant des coupes tissulaires de cryostats.

11. Utilisation d'une substance sélectionnée par un procédé selon la revendication 9 ou 10 dans le traitement de cellules destinées à être transplantées dans un tissu receveur afin de diminuer l'agrégation intravasculaire et/ou d'augmenter le *homing* et/ou d'augmenter la survie des cellules transplantées.

12. Composition pour mettre en oeuvre un procédé selon l'une quelconque des revendications 1 à 11 comprenant des cellules et une substance qui diminue l'adhérence cellulaire, comprenant, en plus, un diluant convenable.

13. Utilisation d'une substance qui diminue l'adhérence cellulaire pour préparer une composition pharmaceutique pour le pré-traitement de cellules destinées à être transplantées afin de diminuer l'agrégation intravasculaire et/ou d'augmenter le *homing* et/ou d'augmenter la survie des cellules transplantées dans le tissu receveur.

14. Utilisation d'une substance qui diminue l'adhérence cellulaire, sélectionnée par un procédé selon la revendication 9 ou 10, pour préparer une composition pharmaceutique pour le pré-traitement de cellules destinées à être transplantées afin de diminuer l'agrégation intravasculaire et/ou d'augmenter le *homing* et/ou d'augmenter la survie des cellules transplantées dans le tissu receveur.

15. Utilisation d'une substance selon la revendication 13 ou 14, dans laquelle lesdites cellules destinées à être transplantées sont allogéniques ou xénogéniques.
